# EUROPEAN PATENT APPLICATION

(11) **EP 4 603 113 A1**
(43) Date of publication of application: **20.08.2025**
(21) Application number: 23877662.9
(22) Date of filing: 11.10.2023
(51) Int. Cl.: A61L 27/44, A61L 27/42, A61L 27/54, A61F 2/28, A61L 27/24, A61L 27/12

(54) **BONE GRAFT MATERIAL AND BONE FORMATION METHOD USING SAME**

(30) Priority: 14.10.2022 KR 20220132100; 14.10.2022 KR 20220132101
(71) Applicant: Y-Bon Bio Inc., Seoul 08742 (KR)
(72) Inventor: PARK, Young Bum, Seoul 03722 (KR); PARK, Jae Han, Seoul 03722 (KR); CHOI, Hyun Min, Seoul 03722 (KR); JUNG, Na Rae, Seoul 03722 (KR)
(74) Representative: Stolmár & Partner Patentanwälte PartG mbB
(86) International application number: PCT/KR2023/015620
(87) International publication number: WO 2024/080737

(57) **Abstract**

The present invention relates to a bone graft material and a bone formation method using same and, more specifically, to a bone graft material and a bone formation method using same, wherein the bone graft material is designed such that, by impregnating a BMP-2 solution into a shield film that covers the upper surface of a bone defect and separates the bone defect from the outside so as to form a bone cover of the bone defect, a filler at the bottom of the shield film can sufficiently form bone in the cover.

## Description

### [Technical Field]

The present invention relates to a bone graft material useable in the medical field, such as bone grafting and bone regeneration, and a bone formation method using the same.

### [Background of Invention]

Bone defects can be treated by a method of inducing bone regeneration and reconstruction in addition to self-healing. If a bone volume is insufficient, bone grafting is performed to increase an amount of bone tissues in the affected area. Forming new bone through bone grafting is the field of tissue engineering. Three major elements of the tissue engineering are source of cells, matrix or scaffold, and signal molecule. In an aspect of bone regeneration, these can be viewed as bone forming cells, a matrix with osteoconductive effects, and a hormone or growth factor with osteoinductive effects, respectively.

Types of bone grafting may include autologous bone grafting, xenogeneic bone grafting, and synthetic bone grafting, etc. Autologous bone grafting is considered the basis for bone transplantation, but it has the disadvantage of being prone to resorption and requiring additional surgery on a donor site. Since xenogeneic bone grafting uses tissues from a different species, there is a risk of immune response and infection. For these reasons, synthetic bone grafting has recently been in the spotlight. Synthetic bone grafting has the advantage of not requiring additional surgery on the donor site and allowing the desired amount to be transplanted.

The surgical process or technology for bone grafting has not yet been completely established. There are occurrences of problems such as the graft material decomposing before bone formation in the defect area is completely achieved, causing an inflammatory reaction, and failing to maintain space sufficiently. Therefore, it is needed to develop new bone graft materials without such problems as described above.

### [Summary of Invention]

### [Problems to be Solved by Invention]

An object of the present invention is to provide a bone graft material capable of effectively inducing bone regeneration in critical-size bone defects that are difficult to heal naturally, as well as a bone formation method using the same.

### [Means for Solving Problems]

1. A bone graft material, including: a shielding film that covers an upper surface of a bone defect and separates the bone defect from an external environment; and a filler that contacts a lower surface of the shielding film and fills the bone defect, wherein the shielding film is impregnated with a BMP-2 (bone morphogenetic protein-2) solution.
2. The bone graft material of the above 1, wherein a concentration of the BMP-2 solution is 0.1 to 1.0 mg/ml.
3. The bone graft material of the above 1, wherein the filler is impregnated with any one selected from the group consisting of fibroblast growth factor (FGF), fibroblast growth factor 2 (FGF-2), platelet-derived growth factor (PDGF), vascular endothelial growth factor (VEGF), transforming growth factor (TGF-beta1), epidermal growth factor (EGF), insulin-like growth factor (IGF), stem cells and exosomes.
4. The bone graft material of the above 1, wherein the filler is impregnated with 0.5 to 1.5 mg/ml of FGF-2 solution.
5. The bone graft material of the above 1, wherein the shielding film has a thickness of 0.1 to 0.5 mm.
6. The bone graft material of the above 1, wherein the shielding film has a porous structure with BMP-2 contained in pores.
7. The bone graft material of the above 1, wherein the shielding film is made of any one selected from the group consisting of collagen, gelatin, alginate, demineralized bone matrix, hyaluronan, deproteinized bone matrix, chitosan, β-tricalcium phosphate, bioglass, hydroxyapatite, calcium phosphate, expanded polytetrafluoroethylene, polymethylethyl methacrylate, polylactic acid, polyglycolic acid, polyurethane, polylactide-co-glycolide, or a combination thereof.
8. The bone graft material of the above 1, wherein the filler is made of any one selected from the group consisting of hydroxyapatite, tricalcium phosphate, biphasic calcium phosphate, bioglass, calcium sulfate, calcium carbonate, calcium phosphate, polyurethane acrylate, polyvinyl alcohol, polyethylene, polypropylene, polyethylene glycol, polylactad-co-glycolide, polycaprolactone, polylactic acid, polyglycolic acid, carboxylic acid, chitosan, isoleucine, ethyl ester, gelatin, collagen, and a combination thereof.
9. A bone formation method, including filling a bone defect with a filler; and forming a shielding film impregnated with a BMP-2 solution on an upper surface of the filler in order to separate the bone defect from an external environment.
10. The bone formation method of the above 9, wherein a concentration of the BMP-2 solution is 0.1 to 1.0 mg/ml.
11. The bone formation method of the above 9, wherein the filler is impregnated with any one selected from the group consisting of fibroblast growth factor (FGF), fibroblast growth factor 2 (FGF-2), platelet-derived growth factor (PDGF), vascular endothelial growth factor (VEGF), transforming growth factor (TGF-beta1), epidermal growth factor (EGF), insulin-like growth factor (IGF), stem cells and exosomes.
12. The bone formation method of the above 9, wherein the filler is impregnated with 0.5 to 1.5 mg/ml of FGF-2 solution.
13. The bone formation method of the above 9, wherein the shielding film has a thickness of 0.1 to 0.5 mm.
14. The bone formation method of the above 9, wherein the shielding film is made of any one selected from the group consisting of collagen, gelatin, alginate, demineralized bone matrix, hyaluronan, deproteinized bone matrix, chitosan, β-tricalcium phosphate, bioglass, hydroxyapatite, calcium phosphate, expanded polytetrafluoroethylene, polymethylethyl methacrylate, polylactic acid, polyglycolic acid, polyurethane, polylactide-co-glycolide, or a combination thereof.
15. The bone formation method of the above 9, wherein the filler is made of any one selected from the group consisting of hydroxyapatite, tricalcium phosphate, biphasic calcium phosphate, bioglass, calcium sulfate, calcium carbonate, calcium phosphate, polyurethane acrylate, polyvinyl alcohol, polyethylene, polypropylene, polyethylene glycol, polylactad-co-glycolide, polycaprolactone, polylactic acid, polyglycolic acid, carboxylic acid, chitosan, isoleucine, ethyl ester, gelatin, collagen, and a combination thereof.

### [Advantageous effects]

The bone graft material of the present invention can effectively induce bone regeneration in bone defects with a critical size or larger, which are difficult to heal naturally.

The bone graft material of the present invention is economical and has reduced side effects when using BMP-2.

The bone graft material of the present invention forms a bone cover on the bone defect that separates the internal area and the external environment of the bone defect, by impregnating the shielding film with BMP-2 solution, thereby allowing the filler containing growth factors and various substances to contribute to the regeneration of bone for a sufficient period of time.

The bone graft material of the present invention enables effective bone regeneration since growth factors and various substances contributing to soft tissue production, such as FGF-2, are included in the filler.

### [Brief Description of Drawings]

FIG. 1 illustrates an example of the bone graft material of the present invention.
FIG. 2 is a schematic diagram of the distribution of surgeries and experimental groups in Experiment A of the present invention.
FIG. 3 is a schematic diagram of the distribution of surgeries and experimental groups in Experiment B of the present invention.
FIG. 4 is a photograph of GBR performed on the rabbit calvaria using collagen membrane, BCP, and rhBMP-2 in the examples herein.
FIG. 5 is a photograph of samples collected after 2, 4 and 8 weeks in the examples herein.
FIG. 6 is a graph showing the healing period and new bone formation by group in Experiment A of the present invention.
FIG. 7 graphically depicts the accumulation of histological components in each group of Experiment A of the present invention.
FIGS. 8A and 8B show HE-stained specimens of each group 2 weeks after healing in Experiment A of the present invention (left original magnification x 12.5, right original magnification x 40). Blue circles, yellow stars, and green triangles represent new bone, bone graft material residue, and connective tissue in the mid-healing phase, respectively.
FIGS. 9A and 9B show HE-stained specimens of each group 4 weeks after healing in Experiment A of the present invention (left original magnification x 12.5, right original magnification x 40). Blue circles, yellow stars, and green triangles represent new bone, bone graft material residue, and connective tissue in the late healing stages, respectively.
FIGS. 10A and 10B show HE-stained specimens of each group 8 weeks after healing in Experiment A of the present invention (left original magnification x 12.5, right original magnification x 40). Blue circles, yellow stars, and green triangles represent new bone, bone graft material residue, and connective tissue in the late healing stages, respectively.
FIG. 11 shows histological samples of each experimental group after a healing period of 2-4-8 weeks in Experiment B of the present invention. New bone: black arrowhead, new bone expanded from existing bone: white arrowhead, bone graft material residue: white arrow, scale bar: 500 µm.
FIG. 12 is a graph comparing new bone formation between experimental groups during the 2-4-8 week healing period of Experiment B of the present invention.
FIG. 13 is a cumulative graph showing the histological components of each experimental group in Experiment B of the present invention.
FIGS. 14A and 14B show RM Pentachrome-stained specimens of each group 2 weeks after healing in Experiment A of the present invention (left original magnification x12.5, right original magnification x 40). Immature new bones are darker red than old bones.
FIGS. 15A and 15B show RM Pentachrome-stained specimens of each group 4 weeks after healing in Experiment A of the present invention (left original magnification x 12.5, right original magnification x 40).
FIGS. 16A and 16B show RM Pentachrome-stained specimens of each group 8 weeks after healing in Experiment A of the present invention (RM Pentachrome Stain; left original magnification x 12.5, right original magnification x 40).
FIG. 17 shows histological samples of each experimental group after a healing period of 2-4-8 weeks in Experiment B of the present invention. Immature new bone: black arrowhead, mature new bone: white arrow, bone graft material residue: white arrow, scale bar: 500 µm.
FIGS. 18A and 18B show specimens stained with anti-osteocalcin antibodies for each group 2 weeks after healing in Experiment A of the present invention (left original magnification x 12.5, right original magnification x 40). Yellow arrows indicate osteocalcin as an indicator of cellular activity for bone formation.
FIGS. 19A and 19B show specimens stained with anti-osteocalcin antibodies for each group 4 weeks after healing in Experiment A of the present invention (left original magnification x 12.5, right original magnification x 40). Yellow arrows indicate osteocalcin as an indicator of cellular activity for bone formation.
FIGS. 20A and 20B show specimens stained with anti-osteocalcin antibodies for each group 8 weeks after healing in Experiment A of the present invention (left original magnification x12.5, right original magnification x 40). Yellow arrows indicate osteocalcin as an indicator of cellular activity for bone formation.
FIG. 21 shows histological samples of experimental group 2 after a healing period of 2-4-8 weeks in experiment B of the present invention. Scale bar: low magnification 1000 µm, high magnification 500 µm.
FIG. 22 illustrates the results of new bone formation according to the group and period in Example 1.
FIG. 23 shows a three-dimensional image of new bone for each group in Example 1.
FIG. 24 shows HE-stained specimens of each group 4 weeks after healing in Example 1.

### [Mode for Carrying out Invention]

The present invention relates to bone graft materials and bone formation methods using the same.

The present invention provides a bone graft material designed such that, by impregnating a shielding film, which covers the upper surface of a bone defect and separates the bone defect from the external environment, with a BMP-2 solution to form a bone cover for the bone defect, a filler on the lower surface of the shielding film can sufficiently form bone within the bone cover, as well as a bone formation method using the same.

The present invention provides a bone graft material that includes a shielding film, which covers an upper surface of a bone defect and separates the bone defect from the external environment, and a filler in contact with the lower surface of the shielding film and filling the bone defect, wherein the shielding film is impregnated with a BMP-2 solution.

The bone graft material of the present invention contains BMP-2 in the shielding film rather than the filler. BMP-2 is a growth factor belonging to the BMP family and is involved in osteogenesis and chondrogenesis.

The shielding film covers the upper surface of the bone defect and separates the bone defect from the external environment. The upper surface of the bone defect refers to the surface where the filler comes into contact with the connective tissue around the defect when the bone defect is filled with the filler. The upper surface of the bone defect may include a flat or curved surface depending on the three-dimensional shape of the defect.

When there are multiple bone defects, the direction of the upper surface of each defect may be different. For example, in the case of a bone defect penetrating the bone, both directions of the bone defect may become upper surfaces, respectively.

The shielding film may cover the upper surface of the bone defect and separate the inside of the bone defect that requires induction of bone formation from the surrounding connective tissue on the outside.

The shielding film is impregnated with BMP-2, wherein BMP-2 is released onto the upper part of the bone defect during the healing period after bone transplantation. Due to the impregnation with BMP-2, BMP-2 is slowly released during the healing period.

As BMP-2 promoting osteogenesis induction is released onto the upper surface of the bone defect, bone formation is induced in the upper part of the bone defect before progressing into the inside of the bone defect. The bone formed first on the upper part of the bone defect supports the shielding film to prevent collapse while protecting the inside of the bone defect. Through this, the filler may form qualitatively superior bone inside the bone defect and achieve a high bone formation rate.

The bone graft material of the present invention may contain BMP-2 only in the shielding film that covers the surface of the bone defect, thereby creating a time difference in bone formation on the upper part and inside of the bone defect.

A concentration of BMP-2 impregnated in the shielding film may range from 0.1 to 1 mg/ml, preferably 0.3 to 0.7 mg/ml, and more preferably 0.4 to 0.6 mg/ml or 0.45 to 0.55 mg/ml. This is a lower concentration than the BMP-2 concentration approved for clinical use for therapeutic purposes, which is 1.5 g/ml.

The bone graft material of the present invention may achieve excellent bone formation effects on the upper part of the bone defect using a low-concentration BMP-2 solution within the range of 0.1 to 1 mg/ml.

The bone graft material of the present invention may induce bone formation more economically and effectively using a low-concentration BMP-2 solution. Further, there are no side effects such as hematoma, severe edema, difficulty in swallowing, or excessive bone formation caused by the conventional high-concentration BMP-2 solution.

The material of the shielding film is not particularly limited as long as it can be impregnated with the BMP-2 solution and is permeable.

The shielding film may be made of any one selected from the group consisting of collagen, gelatin, alginate, decalcified bone matrix, hyaluronan, deproteinized bovine bone matrix, chitosan, β-Tricalcium phosphate (β-TCP), bioglass, hydroxyapatite (HA), calcium phosphate, expanded polytetrafluoroethylene (ePTFE), polymethylethyl methacrylate, polylactic acid (PLA), polyglycolic acid (PGA), polyurethane, polylactide-co-glycolide (PLGA), or a combination thereof.

The shielding film may have a porous structure. A size of pores in the shielding film may be appropriately selected by a person skilled in the art who considers physical properties of the shielding film. For example, the pore size may range from, but is not limited to, 1 to 100 µm, 1 to 70 µm, 1 to 50 µm, 1 to 30 µm, 1 to 20 µm, 5 to 100 µm, 5 to 70 µm, 5 to 50 µm, 5 to 30 µm, 5 to, 20 µm, 10 to 100 µm, 10 to 70 µm, 10 to 50 µm, 10 to 30 µm, or 10 to 20 µm.

The pore size of the shielding film may be selected to be smaller than the pore size of the filler.

It is preferable that the shielding film is made of a sustained-release preparation that can continuously release the BMP-2 solution.

The shielding film may have a multi-layer structure of two or more layers. At this time, each layer may be different in material, pore diameter, porosity, structure, etc. For example, the shielding film may include a triple layer structure of an upper layer, a middle layer and a lower layer. The upper or lower layer is made of a structure or material with lower permeability than the middle layer, which may relatively confine the BMP-2 solution inside and suppress instant release. The upper or lower layer may be pore-free or contain no pores larger than 1 nm.

The shielding film may include a multi-layered thin film structure.

The method of impregnating BMP-2 into the shielding film is not limited to a specific method. For example, BMP-2 may be impregnated into the shielding film by immersing the shielding film in a BMP-2 solution or injecting the BMP-2 solution into the shielding film.

A solvent of the BMP-2 solution is not particularly limited as long as it does not inhibit the physiological activity of BMP-2, and may be, for example, saline solution or distilled water.

The shielding film may be a material that is absorbable or non-absorbable to the tissue of the bone defect. In the case of absorbable materials, the absorption period in the bone defect may be 3 months or more, 4 months or more, 5 months or more, 6 months or more, 7 months or more, 8 months or more, otherwise, 12 months or less, 11 months or less, 10 months or less, 9 months or less, 8 months or less, 7 months or less, 6 months or less, 5 months or less, or 4 months or less.

By manufacturing the shielding film from a material that does not decompose in a short period of time, the internal area and the external environment of the bone defect may be spatially separated by the shielding film for a sufficient period of time for bone formation. As the inside of the bone defect is separated from the external environment, bone formation may sufficiently proceed inside the bone defect.

An area and thickness of the shielding film may be appropriately selected by a person skilled in the art considering a shape of the bone defect, a degree of defect, the material of the shielding film, etc.

The area of the shielding film may be larger than the upper surface of the bone defect so as to facilitate adhesion to the bone defect. The shielding film may be fixed to the bone defect with an abutment, etc., if necessary.

A shielding film may have a thickness of 0.1 to 0.5 mm, 0.2 to 0.5 mm, or 0.3 to 0.5 mm. If the shielding film has the above thickness, it is easy to impregnate the BMP-2 solution and maintain a physical shape of the shielding film, but it is not limited thereto.

The type of bone formed is not particularly limited and includes alveolar bone.

The bone graft material of the present invention may include a filler that contacts a lower surface of the shielding film and fills the bone defect.

The filler may mechanically support the shielding film by filling the bone defect in contact with the lower surface of the shielding film and suppress initial release of BMP-2 released from the shielding film.

The filler may maintain the volume of the bone defect during the bone regeneration period. The filler is preferably one that is easy to mimic the shape of the defect and is similar to the extracellular matrix of bone.

The material of the filler is not particularly limited, and may be autologous bone, allogeneic bone, xenograft bone, or synthetic bone.

The filler may be made of any one, but it is not limited thereto, selected from the group consisting of hydroxyapatite (HA), tricalcium phosphate (TCP), biphasic calcium phosphate (BCP), bioglass, calcium sulfate, calcium carbonate, calcium phosphate, polyurethane acrylate (PUA), polyvinyl alcohol (PVA), polyethylene (PE), polypropylene (PP), polyethylene glycol (PEG), polylactide-co-glycolide (PLGA), polycaprolactone (PCL), polylactic acid (PLA), polyglycolic acid (PGA), carboxylic acid, chitosan, isoleucine, ethyl ester, gelatin, collagen, and a combination thereof, but it is not limited thereto.

Biphasic calcium phosphate (BCP) is one of bone graft materials, which is prepared of a mixture of hydroxyapatite (HA) and β-tricalcium phosphate (TCP). A weight ratio of HA and β-TCP in BCP may be, for example, 30 : 70 to 70 : 30, preferably 30 : 40 to 70 : 60, but it is not limited thereto.

In one embodiment, the bone graft material of the present invention may include at least one growth factor and various substances in the filler. The growth factor and various substances may be any one selected from the group consisting of fibroblast growth factor (FGF), fibroblast growth factor 2 (FGF2), platelet derived growth factor (PDGF), vascular endothelial growth factor (VEGF), transforming growth factor beta 1 (TGF-beta1), epidermal growth factor (EGF), insulin-like growth factor (IGF), stem cells and exosomes.

In one embodiment, the bone graft material of the present invention may include a filler impregnated with an FGF-2 solution.

FGF-2 is a growth factor belonging to the FGF family that binds to the fibroblast growth factor receptor (FGFR) protein. It is involved in angiogenesis and may help bone regeneration by inducing bone marrow-derived mesenchymal cells and promoting osteoblast differentiation.

The bone graft material of the present invention may combine a shielding film impregnated with BMP-2 and a filler impregnated with an FGF-2 solution, so as to improve the formation of new bone in the upper bone in the early stages of treatment, and further bone regeneration effect in the defect area.

A concentration of the FGF-2 solution may be 0.5 to 1.5 mg/ml, 0.7 to 1.3 mg/ml, 0.8 to 1.2 mg/ml, 0.9 to 1.1 mg/ml, or 0.95 to 1.05 mg/ml.

In one embodiment, the bone graft material of the present invention may not include BMP-2 in the filler. The bone graft material of the present invention includes BMP-2 only in the shielding film that covers the surface of the bone defect, thereby creating a time difference in bone formation on the upper part and inside of the bone defect.

The present invention provides a bone formation method, including the steps of: filling a bone defect with a filler; and forming a shielding film impregnated with a BMP-2 solution on an upper surface of the filler to separate the bone defect from the external environment.

The bone formation method of the present invention is a guided bone regeneration (GBR) method as a dental bone regeneration technique, which can preserve a space for bone formation by applying a shielding film to prevent surrounding soft tissues from being formed faster than bone tissues. Existing guided bone regeneration techniques have focused on applying various bone graft materials and growth factors to the inside of bone defects for bone regeneration, however, the bone formation method of the present invention is not restricted to demonstrating the effect of a combination of bone graft materials and growth factors but focuses on creating an environment favorable to internal bone formation by strengthening the function of the shielding film.

The bone formation method of the present invention may apply rhBMP-2 to the shielding film rather than to the bone defect. rhBMP-2 has a bone formation-inducing function. When rhBMP-2 is applied to bone defects, post-operative complications such as severe swelling, pain, and non-specific bone formation may occur. Further, temporary and rapid release of high-concentration, high-dose growth factors may become the main cause of these complications. In the bone formation method of the present invention, such problems do not occur by applying rhBMP-2 to the shielding film.

With regard to matters related to the composition and effects of the bone graft material in the bone formation method of the present invention, the previous description of the bone graft material is directly applied.

The present invention provides a method for treatment of bone diseases, based on the above bone formation method.

Hereinafter, the present invention will be described in more detail by way of examples.

### Example 1

### 1-1. Experimental animals and materials

### (1) Laboratory animals

Ten 6-week-old SD rats weighing 250-300 g were prepared. The animals were quarantined and acclimatized for 1 week and then fed a standard laboratory diet. Raising, management, and surgical procedures followed the animal testing standards of the Yonsei Medical Center Animal Care Committee (approval number 2022-0247).

### (2) Materials

### 1) Alginate/Gelatin gel

Alginate/Gelatin gel was prepared by diluting alginic acid sodium salt from brown algae (Sigma-Aldrich, USA) and gelatin from porcine skin (Sigma-Aldrich, USA) powder in PBS at a ratio of 6:10.

### 2) 3D printing PCL

Polycaprolactone (Corbion N.V., Netherlands) is a biodegradable polymer that hydrolyzes in the body, and a scaffold with upper, lower, and side voids was produced by 3D printing using a raw material having an intrinsic viscosity of 1.19 dl/g.

### 3) BMP-2

As BMP-2, rhBMP-2 (GENOSS, Suwon, Korea) derived from E. coli was used. The rhBMP-2 solution at a concentration of 0.5 mg/ml was applied together with a total volume of PBS when producing the Alginate/Gelatin gel.

### 1-2. Experimental design

Total three groups were set up as shown in Table 1. Bone graft material for each group was implanted subcutaneously on the back of SD rats. The healing period was set to 2 weeks and 4 weeks. The control group was implanted with only PCL, a filler, and experimental group 1 was implanted with Alginate/Gelatin gel combined on the upper part of the PCL as a shielding film. In experimental group 2, Alginate/Gelatin gel impregnated with rhBMP-2 solution at a concentration of 0.5 mg/ml was applied as a shielding film and grafted onto the upper part of the PCL.

**[TABLE 1]**

| Experiment | Bone graft material | |
|---|---|---|
| | Shielding film | Filler |
| Control group | - | PCL |
| Experimental group 1 | Alginate/Gelatin gel | PCL |
| Experimental group 2 | Alginate/Gelatin gel (rhBMP-2, 0.5 mg/ml) | PCL |

### 1-3. Experiment result

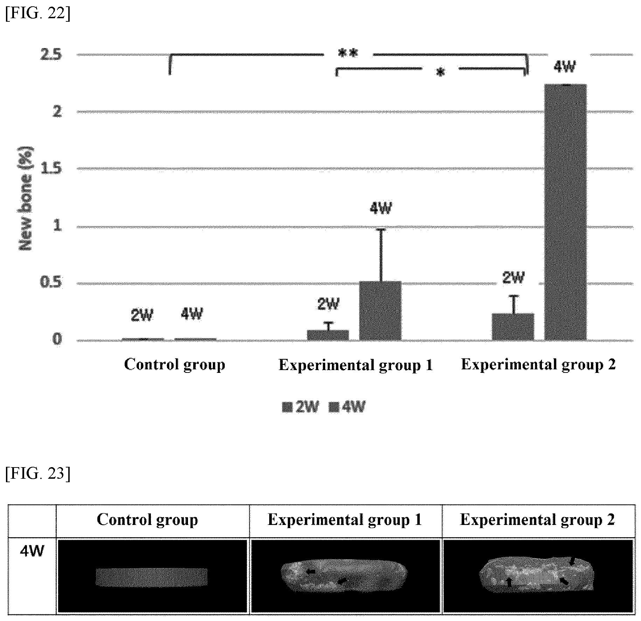

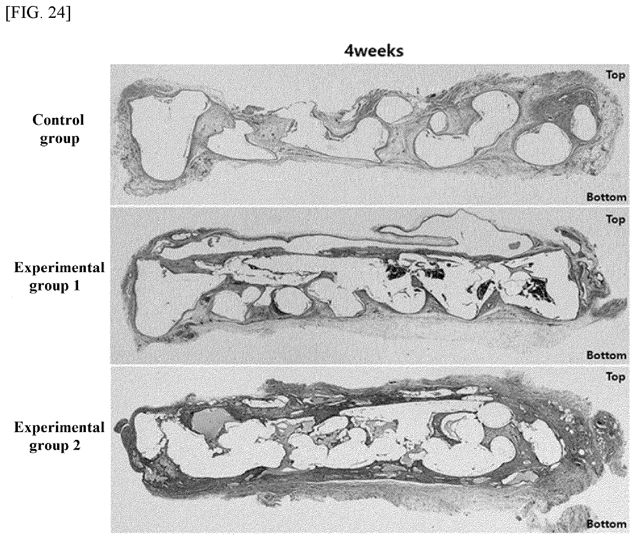

The new bone formation rate was significantly higher in the group in that Alginate/Gelatin gel and PCL impregnated with rhBMP-2 was applied at a concentration of 0.5 mg/ml (FIG. 22). As shown in FIGS. 23 and 24, it was confirmed that new bone was intensively formed on the upper part of the bone graft material to which rhBMP-2 was applied during the 4w healing period. Further, the bone formation method of the present invention could be used as rapid guided bone regeneration (rGBR), as a new procedure that preserves bone defects by applying rhBMP-2 to the shielding film rather than the defect area so as to quickly form a natural bone wall.

### Example 2

### 2-1. Experimental animals and materials

### (1) Laboratory animals

54 New Zealand white rabbits, aged 16-20 weeks and weighing 3-3.5 kg, were prepared. The animals were quarantined and acclimatized for 1 week and then fed a standard laboratory diet. Raising, management, and surgical procedures followed the animal testing standards of the Yonsei Medical Center Animal Care Committee (approval number 2016-0062).

### (2) Materials

### 1) Collagen membrane

High purity, type-I, 300 µm thick collagen membrane derived from bovine tendon was used (Genoss, Suwon, Korea). The collagen membrane is designed to last more than six months in the human body. The collagen membrane had a dense multilayer structure with no pores on the top or bottom surface.

### 2) BCP

Osteon II (Genoss, Suwon, Korea) was used as BCP. Osteon II has a weight ratio of hydroxyapatite (HA) and β-tricalcium phosphate (TCP) of 30:70, pores of 250 µm, porosity of 70%, and particle size of 0.5-1.0 mm. The appropriate dose for an 8 mm diameter bone defect in the rabbit calvaria was 70 mg, and the same dose was used for each bone defect.

### 3) BMP-2

BMP-2 was used as rhBMP-2 (GENOSS, Suwon, Korea) derived from E. coli. rhBMP-2 solutions with concentrations of 0.5 and 1.0 mg/ml were prepared by diluting 25 or 50 µg of rhBMP-2 with 50 µl of saline, respectively. The control group used only 50 µl of physiological saline.

### 4) FGF-2

FGF-2 was used as rhFGF-2 (derived from E. coli, GENOSS, Suwon, Korea). FGF-2 solutions at concentrations of 0.5 mg/ml and 1.0 mg/ml were prepared by diluting them with saline solution. BCP was immersed in 50 µl of FGF-2 dilution solution at each concentration, and 25 µg and 50 µg of rh-FGF2 were added, respectively. The control group used only 50 µl of physiological saline solution.

### 2-2. Experimental design

### (1) Experiment A

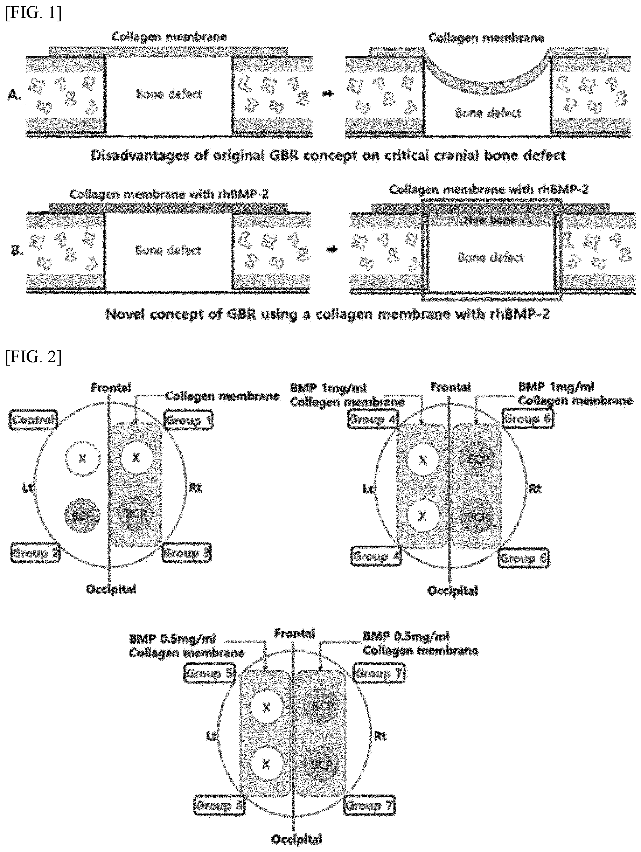

In Experiment A, total 8 groups were set as shown in Table 2. In each of 30 rabbits, four bone defects were created in the calvaria (FIG. 2). To prevent them from influencing each other during the surgical process, only one rhBMP-2 concentration group was set per rabbit. Healing periods were set to 2, 4 and 8 weeks. Ten animals/period were used. Twelve rabbits were used in the control group, while using 9 per each of experimental groups 1 to 3, 9 per each of experimental groups 4 and 5, and 9 per each of experimental groups 6 and 7.

The control group did not receive bone transplantation, experimental group 1 received only the collagen membrane, experimental group 2 received only BCP, and experimental group 3 received both collagen membrane and BCP. Experimental groups 4 and 5 received collagen membranes impregnated with 1.0 and 0.5 mg/ml rhBMP-2, respectively. Experimental groups 6 and 7 received BCP as well as the collagen membrane of experimental groups 4 and 5.

**[TABLE 2]**

| Experiment A | Bone graft material | |
|---|---|---|
| | Shielding film | Filler |
| Control group | - | - |
| Experimental group 1 | Collagen membrane | - |
| Experimental group 2 | - | BCP |
| Experimental group 3 | Collagen membrane | BCP |
| Experimental group 4 | Collagen membrane (rhBMP-2, 1.0 mg/ml) | - |
| Experimental group 5 | Collagen membrane (rhBMP-2, 0.5 mg/ml) | - |
| Experimental group 6 | Collagen membrane (rhBMP-2, 1.0 mg/ml) | BCP |
| Experimental group 7 | Collagen membrane (rhBMP-2, 0.5 mg/ml) | BCP |

### 2) Experiment B

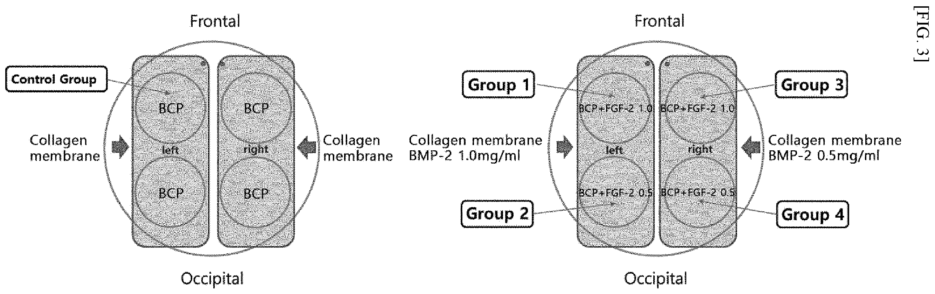

In Experiment B, total 5 groups were set as shown in Table 3. Bone defects were formed in 24 rabbits as in Experiment A. The four concentration groups were arranged in the four bone defects of each individual to reduce individual specificity. Since it is difficult to fix four collagen membranes at each concentration to prevent interference with each other during surgery, only two BMP-2 concentration groups per subject were set in each of the two bone defects on the left and right (FIG. 3). Healing periods were set to 2, 4 and 8 weeks. Eight rabbits/period were used.

**[TABLE 3]**

| Experiment B | Bone graft material | |
|---|---|---|
| | Shielding film | Filler |
| Control group | Collagen membrane | BCP |
| Experimental group 1 | Collagen membrane (rhBMP-2, 1.0 mg/ml) | BCP (FGF-2, 1.0 mg/ml) |
| Experimental group 2 | Collagen membrane (rhBMP-2, 1.0 mg/ml) | BCP (FGF-2, 0.5 mg/ml) |
| Experimental group 3 | Collagen membrane (rhBMP-2, 1.0 mg/ml) | BCP (FGF-2, 1.0 mg/ml) |
| Experimental group 4 | Collagen membrane (rhBMP-2, 1.0 mg/ml) | BCP (FGF-2, 0.5 mg/ml) |

The control group used collagen membrane and BCP without applying BMP-2 or FGF-2. Experimental groups 1 to 4 used the shielding film and filler of the control group, but the impregnation concentrations of BMP-2 and FGF-2 were different.

### (3) Surgical protocol

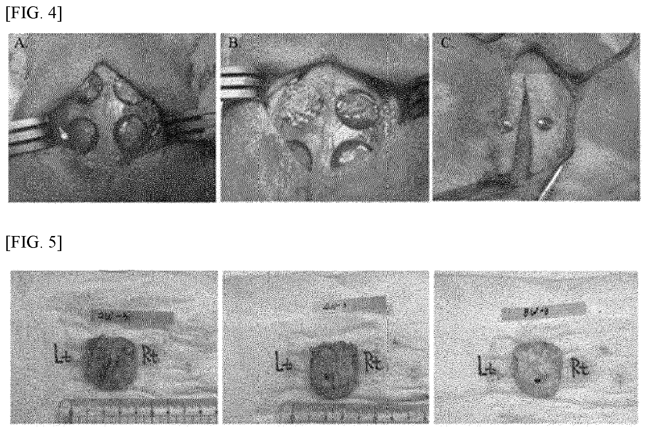

Rabbits were anesthetized with subcutaneous injection of 1.5 mg/kg Zolazopam (Zoletil, Virbac Korea Co., Seoul, Korea) and intramuscular injection of 5 mg/kg Xylazine HCl (Rompun, Bayer Korea Co., Seoul, Korea). Then, 10 minutes later, the skin of the calcaria was disinfected with povidone-iodine, and local anesthesia was induced with 2% lidocaine containing 1:80,000 epinephrine (lidocaine HCl, Huons, Seongnam, Korea). A 2.0-2.5 cm incision was made in the skin and periosteum from the anterior part of the frontal bone via the parietal bone to the anterior part of the occipital bone, and the periosteum was lifted with a full-thickness flap. Four bone defects were formed in a square shape on the exposed surface of the calvaria using a trephine bur at a minimum interval of 3 mm (Mr. Curette Tech, Seongnam, Korea). The defect was circular with 1 to 2 mm depth and 8 mm in outer diameter, extending to the inferior cortical bone without damage to the dura. Bone fragments created by defect formation were gently extracted using a curette. It was confirmed that the dura was intact and there was no bleeding. Then, rhBMP-2 dilution, 10 × 20 mm² collagen membrane, and saline solution were prepared appropriately for each group. The collagen membranes were then soaked in 50 µl rhBMP-2 solution for 30 min as appropriate for each group. Collagen membranes for groups 1 and 3 were immersed in 50 µl of physiological saline. Then, BCP was appropriately implanted into each bone defect, and a collagen membrane was placed over the bone defect and fixed with micro pins (FIG. 4). The periosteum was first sutured with 4-0 Vicryl absorbable suture (Ethicon, Somerville, NJ, USA), and then the epidermis was sutured.

### (4) Laboratory animal sacrifice

Rabbits were sacrificed by intravenous injection of concentrated sodium pentobarbital after a healing period of 2, 4 or 8 weeks. The epidermis was incised and the periosteum was separated. Samples were collected by removing marginal bone using a handpiece while maintaining a sufficient distance from the bone defect. The front side of the specimen was marked.

### (5) Histological and histomorphological analysis

### 1) Experiment A

Specimens were fixed in 10% formalin for 6 weeks, washed, and desalted in 2.5% NaOCl and 17% EDTA solution for 18 days. Dehydration and paraffin infiltration were performed using ethanol and xylene. After embedding, the specimens were sliced to a thickness of 3-4 µm. Slices were then cut in an anterior and posterior direction along the sagittal plane passing through the center of the bone defect. Hematoxylin-eosin (HE) and Russell-Movat Pentachrome staining (American MasterTech, USA) were performed and tissue slides were prepared. Specimens were photographed at 12.5× and 40× magnification using a BX50 light microscope (Olympus, Tokyo, Japan), and histological measurements were performed using HE-stained tissue slides. New bone (NB), remaining bone graft material (RB), and fibrovascular connective tissue (FCT), and shrinkage volume (SV) within the defect area were measured using Image-pro Plus software (Media Cybernetics, Silver Spring, MD, USA). Each area was described as a percentage of the total defect area. Russell-Movat Pentachrome staining is a tissue slide and tissue-specific staining method that identifies where tissues are located during development. The degree of osteogenesis and new bone differentiation were analyzed.

### 2) Experiment B

Specimens were imaged using an optical microscope (Leica DM 2500, Leica Microsystems, Wetzlar, Germany) at 12.5× and 40× magnification, and histological measurements were performed using HE-stained tissue slides. The new bone area and remaining bone graft area were measured using Image-pro plus (Media Cybernetics, Silver Spring, Maryland, USA). The area of new bone and the area of remaining bone graft material were calculated as a percentage of the total area.

Bone development was observed on tissue slides using Russel-Movat Pentachrome staining (American MasterTech) as a tissue-specific staining method according to tissue development, and differentiation of new bone was analyzed (bone: dark yellow, new bone: red, cartilage: green).

### (6) Immunohistochemical analysis

Anti-osteocalcin antibody (OCG3) immunohistochemical staining was performed to determine the vascularity and density of osteoblasts. Anti-osteocalcin antibody (abcam, ab13420, 1/150) and anti-mouse HRP (abcam, ab205719, 1/150) were used according to the manufacturer's instructions.

### (7) Statistical analysis

### 1) Experiment A

Out of 200 samples, 3 failed during sampling. Among 117 samples, 17 were excluded as outliers while 100 were retained for analysis. Statistical analysis was performed using histological data. Normality was confirmed using the Shapiro-Wilk test. Data are presented as mean and standard deviation.

Two-way ANOVA was performed to determine whether there was a difference in bone formation rate according to the treatment period by group. Post hoc analysis (LSD method) was performed to determine whether there were differences between treatment groups. Statistical calculations were performed using SPSS version 25.0 (IBM Corp., Armonk, NY, USA). Statistical significance was set to 5% and a p-value < 0.05 was considered statistically significant.

### 2) Experiment B

Among 58 samples, 56 samples were used for analysis, excluding two outliers. The statistical analysis method is the same as Experiment A.

### 2-3. Experiment result

### (1) Anatomical results

### Experiment A

Four bone defects were formed in each of 30 rabbits. After 2, 4 and 8 weeks, rabbits were sacrificed and tissue samples were collected (FIG. 5). Three samples from the control group at weeks 4, 6 and 8 were damaged during the sample extraction process and were excluded from the analysis. There are differences in defect healing depending on the healing period of 2, 4 and 8 weeks. In the 2nd week sample, the border of the defect was clearly defined, while the border of the defect was less clear from the 4th to 8th week.

### (2) Histological results (H-E staining)

### Experiment A

### 1) Histological sample analysis 2 weeks after healing

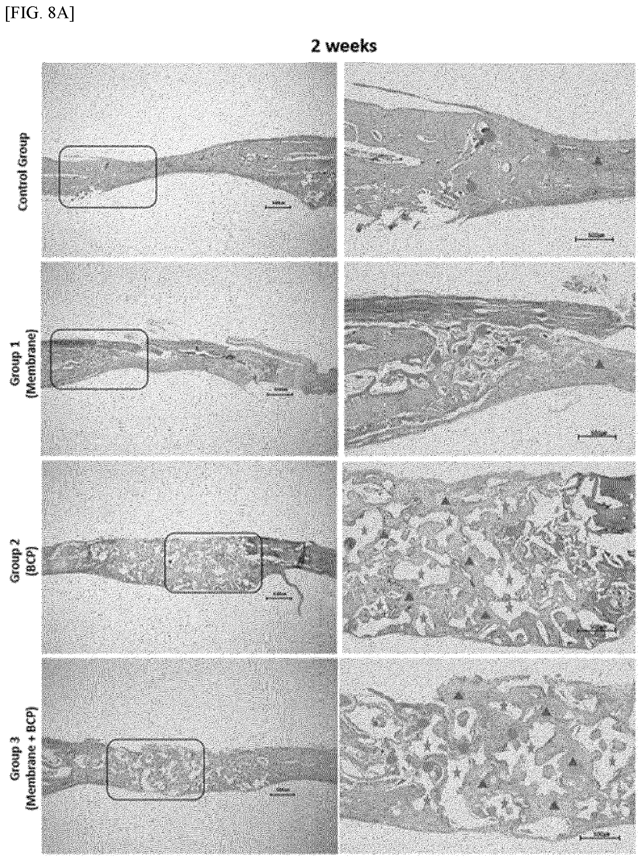

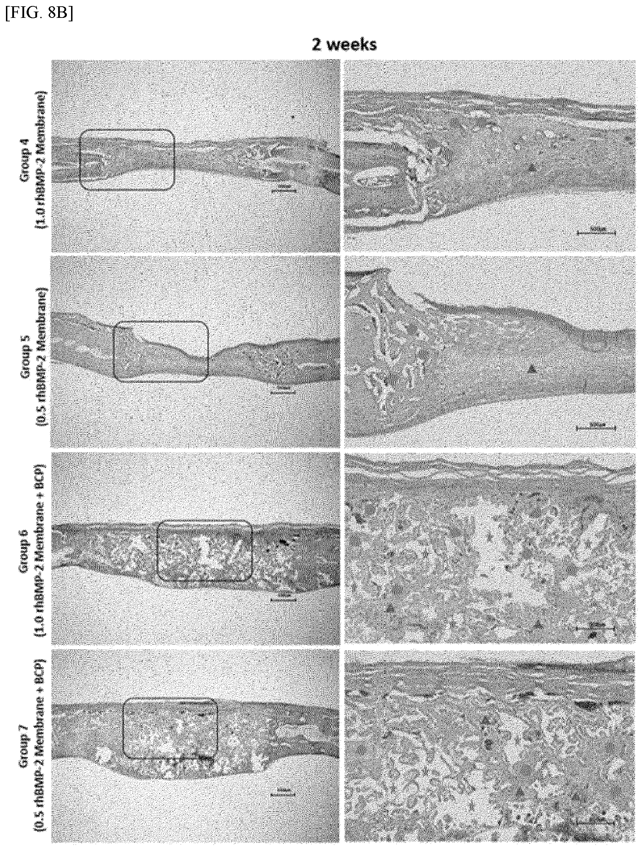

FIG. 8 shows images of H-E stained tissue specimens 2 weeks after healing. In the control group, new bone was formed only near the defect border. The bone was mostly woven bones that originated near existing bones. In the experimental group that received BCP, new bone began to form around the graft material at the boundary between the existing left and right bones. In particular, in the experimental group with collagen membrane and BCP, new bone was formed in the center of the defect. Osteogenesis mainly began in the overlying periosteum. There were high numbers of inflammatory cells and early connective tissue was observed.

### 2) Histological sample analysis 4 weeks after healing

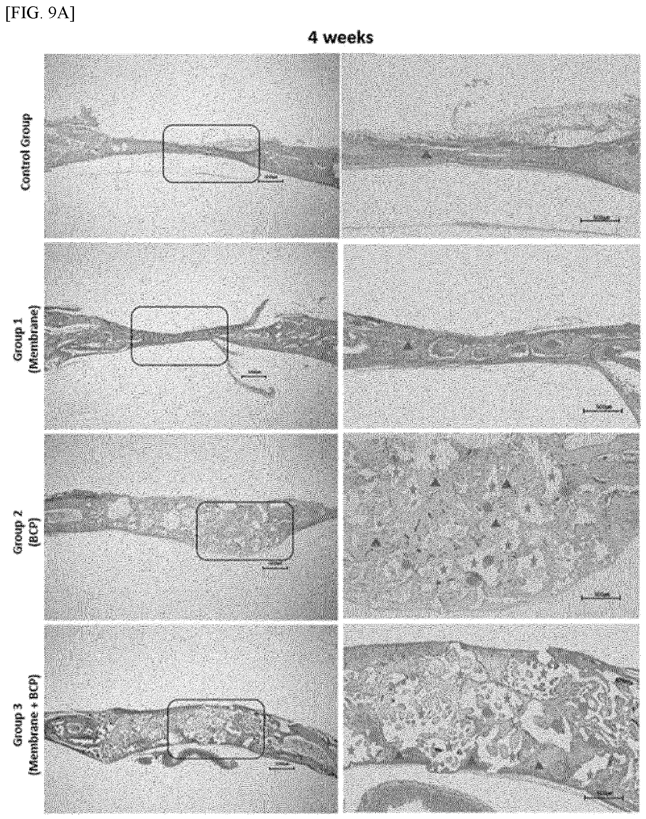

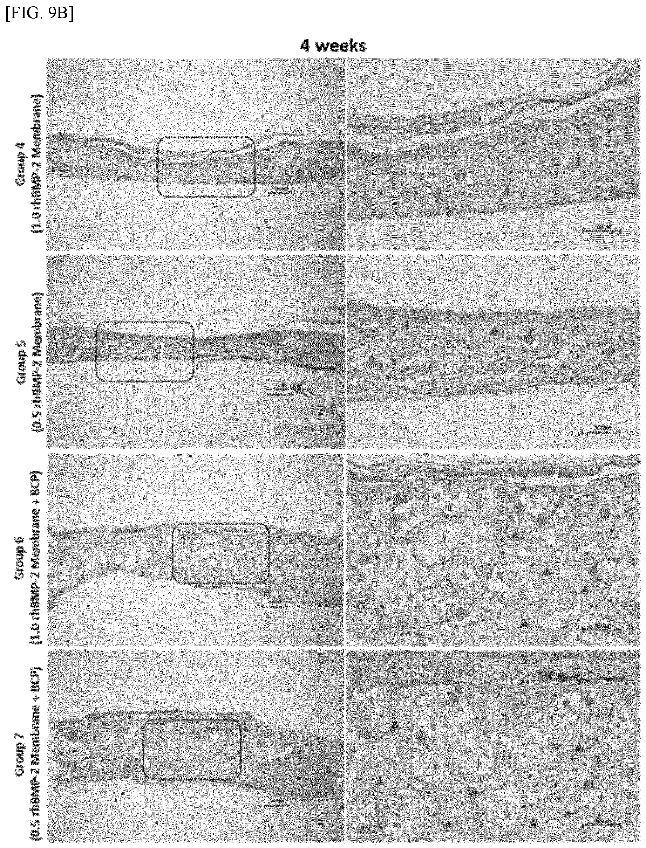

FIG. 9 shows images of HE-stained tissue specimens 4 weeks after healing. In the control group, more fatty tissues were present on the outer side of the upper defect than after 2 weeks. In experimental group 3, new upper bone was formed, loose connective tissue was formed around the remaining bone graft material, and connective tissue consisting of a significant amount of high-density inflammatory tissues was formed around the existing bone. In experimental groups 6 and 7, mature new bone was formed from the existing bone above the defect.

### 3) Histological sample analysis 8 weeks after healing

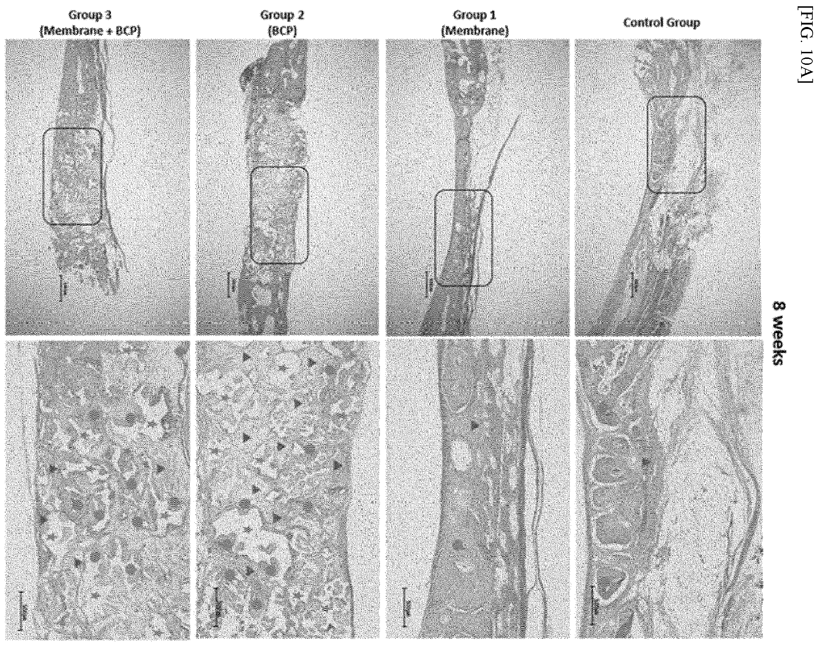

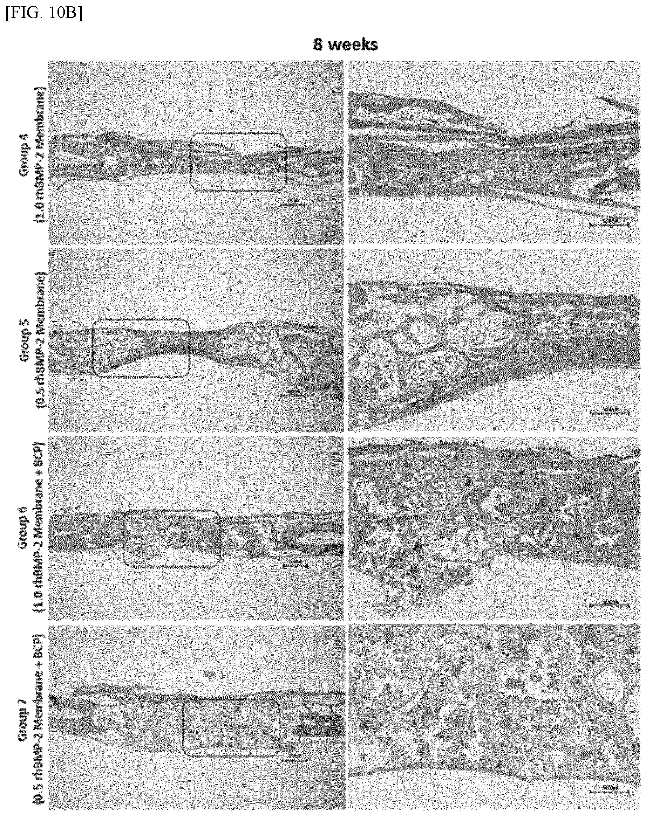

FIG. 10 shows images of H-E stained tissue samples from the control and experimental groups after 8 weeks of healing. The upper periosteum of the control group was completely covered with a layer of fatty tissue, but there was little new bone. In most experimental groups, newer and histologically more mature bone was formed in the center of the defect, and the boundary between new bone and existing bone became less clear. Only in experimental groups 6 and 7, mature new bone was connected to the upper part of the defect. In experimental groups 2, 3, 6 and 7 where BCP was performed, the remaining amount of bone graft material was significantly reduced compared to before. In all groups, only connective tissue was observed in the dura mater and no new bone was observed.

### Experiment B

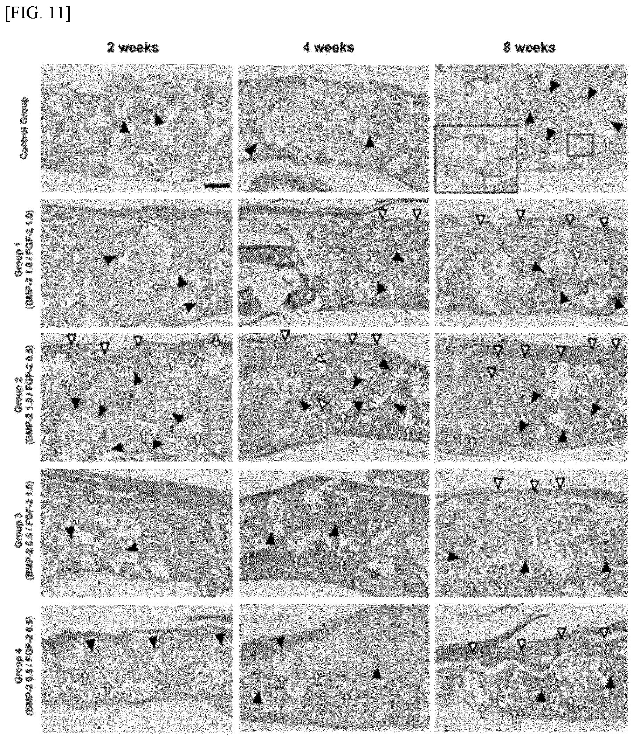

FIG. 11 shows images of H-E stained tissue samples from the control and experimental groups according to the healing process. Two weeks after transplantation, new bone (black arrowhead) begins to form around the existing bone and graft material (white arrow) in both the control and experimental groups. Most of the new bone was in the form of woven bone, but in experimental groups 2 and 3, thin new bone at the top (white arrowhead) derived from existing bone appeared.

In the control group 4 weeks after transplantation, new bone (black arrowhead) and bone graft material (white arrow) were observed around the existing bone and were similar to the control group at 2 weeks. In the experimental group, new bone (white arrowhead) extending from the existing bone was observed at the top, and its thickness increased compared to the 2-week and 4-week groups.

At 8 weeks after transplantation, new bone (arrowhead) continuously covered the upper defect area in all experimental groups, which was not observed in the control group. In the control group, new bone (black arrowhead) was observed around the remaining bone graft material (white arrow) and existing bone. In particular, when the new bone encompassed or surrounded the bone graft material, fatty tissue (black square) was observed inside the same. The experimental group showed a significant decrease in the remaining bone graft material without fatty tissue formation.

### (3) Histomorphological results

### Experiment A

### 1) New bone formation

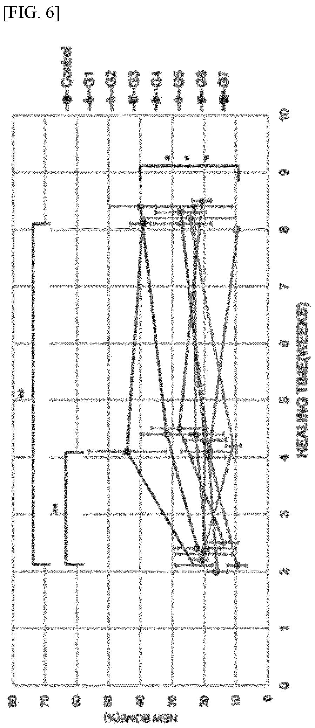

Two-way ANOVA was performed to determine whether there were differences in bone formation rates according to groups and healing periods. As a result, the control group had the lowest bone formation rate while experimental group 7 had the highest bone formation rate. The p-values according to group and healing period were < 0.001 and 0.008, which were smaller than 0.05 (Table 4, FIG. 6). Post hoc analysis using the LSD method confirmed that control and experimental groups 1, 2, 3, 4 and 5 had lower scores than experimental groups 6 and 7. According to the healing period, average bone formation rates of 18.71%, 23.29%, and 27.03%, respectively, were demonstrated. Post hoc analysis using the LSD method showed that the 2-week healing period had a lower bone formation rate than the 4- and 8-week healing periods.

**[TABLE 4]**

| New bone formation analysis of Experiment A | | | | |
|---|---|---|---|---|
| Experiment A | N | NB (%) | p-value | LSD type post hoc analysis |
| Control group | 10 | 14.54± 4.86 | < 0.001 | Control group, experimental groups 1 to 5 < experimental groups 6 & 7 |
| Experimental group 1 | 10 | 18.50± 10.88 | | |
| Experimental group 2 | 11 | 18.31± 10.17 | | |
| Experimental group 3 | 12 | 22.47± 9.02 | | |
| Experimental group 4 | 16 | 21.58± 10.65 | | |
| Experimental group 5 | 15 | 20.30± 7.99 | | |
| Experimental group 6 | 14 | 31.25± 11.62 | | |
| Experimental group 7 | 12 | 32.56± 12.62 | | |

| Healing period | | | | |
|---|---|---|---|---|
| 2 weeks | 37 | 18.71± 7.61 | 0.008 | 2 weeks < 4 weeks, 8 weeks |
| 4 weeks | 31 | 23.29± 12.34 | | |
| 8 weeks | 32 | 27.03± 12.46 | | |
| Value is average ± LSD, tested by two-way ANOVA | | | | |

### 2) Histological composition

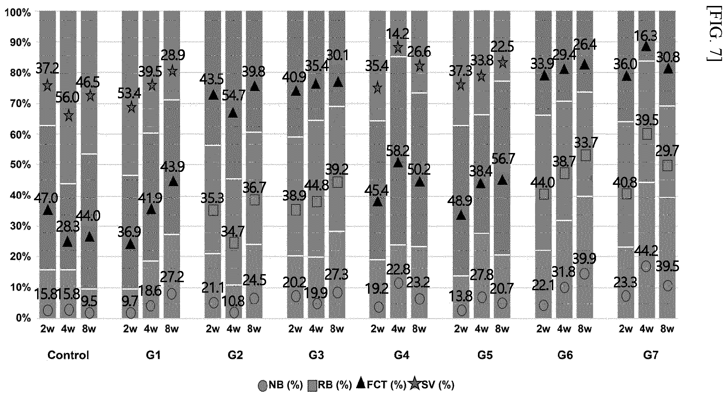

New bone (NB, %), remaining bone graft material (RB, %), fibrovascular connective tissue (FCT, %), and shrinkage volume (SV, %) were measured. Each area was converted to a percentage of the total defect area. FIG. 7 is a graph showing how the four components change in each group according to the healing period. In all groups except the control group, the amount of new bone was greater at 8 weeks than at 2 weeks. Experimental groups 6 and 7 showed significant improvement in new bone formation compared to the other groups. The SV of the group without administration of BCP in experimental groups 4 and 5 was lower than that in the control group and experimental group 1. RB in experimental groups 6 and 7 decreased over time.

### Experiment B

The new bone area and the remaining bone graft material area were measured. Specifically, the area of new bone and the area of remaining bone graft material relative to the total area were calculated as percentages.

### 1) New bone formation

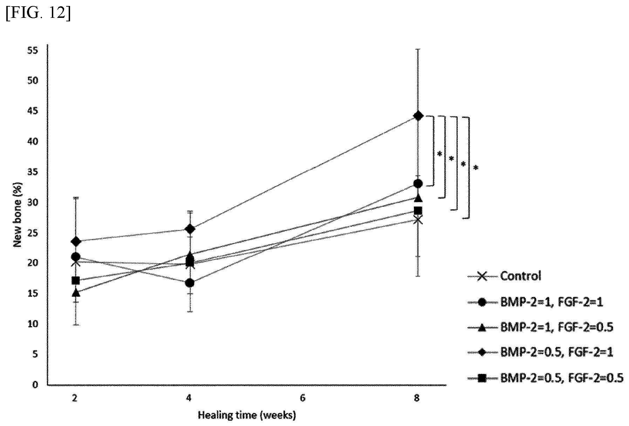

In a two-way ANOVA to determine the difference in bone formation rate according to the concentration combination of BMP-2 and FGF-2 and healing period, the p-values were 0.04 and < 0.001, respectively, which were less than 0.05. Therefore, the difference in bone formation rate according to the combination of BMP-2 and FGF concentrations and healing period was shown at a significance level of 5% (Table 5, FIG. 12).

In view of BMP-2 and FGF-2 concentration combinations, the average bone formation rates were 22.14%, 22.42%, 22.51%, 23.87%, and 30.65% for experimental group 4, control group, experimental group 2, experimental group 1, and experimental group 3, respectively. That is, the BMP-2 0.5 mg/ml and FGF-2 0.5 mg/ml groups showed the lowest bone formation rate, while the BMP-2 0.5 mg/ml and FGF-2 1.0 mg/ml groups showed the highest bone formation rate. In a post hoc analysis using the LSD method, the relationship between groups was as follows: control group = experimental group 1 = experimental group 2 = experimental group 4 < experimental group 3.

The bone formation rates were 19.15%, 20.8% and 32.20% in the order of average healing periods of 2 weeks, 4 weeks and 8 weeks, respectively. In other words, the longer the healing period, the faster the rate of bone formation. In a post hoc analysis using the LSD method, the relationship between healing periods was 2 weeks = 4 weeks < 8 weeks.

**[TABLE 5]**

| New bone formation analysis of experiment B | | | | |
|---|---|---|---|---|
| Experiment B | N | NB (%) | p-value | LSD type post hoc analysis |
| Control group | 12 | 22.42± 9.06 | 0.040 | Control group = G1 = G2 = G4 < G3 |
| Experimental group 1 | 11 | 23.87± 11.23 | | |
| Experimental group 2 | 12 | 22.51± 8.48 | | |
| Experimental group 3 | 10 | 30.65± 11.39 | | |
| Experimental group 4 | 11 | 22.14± 7.35 | | |

| Healing period | | | | |
|---|---|---|---|---|
| 2 weeks | 18 | 19.15± 7.31 | <0.001 | 2 weeks = 4 weeks < 8 weeks |
| 4 weeks | 19 | 20.80± 6.46 | | |
| 8 weeks | 19 | 32.20± 9.56 | | |
| Value is average ± LSD, tested by two-way ANOVA | | | | |

### 2) Histological composition

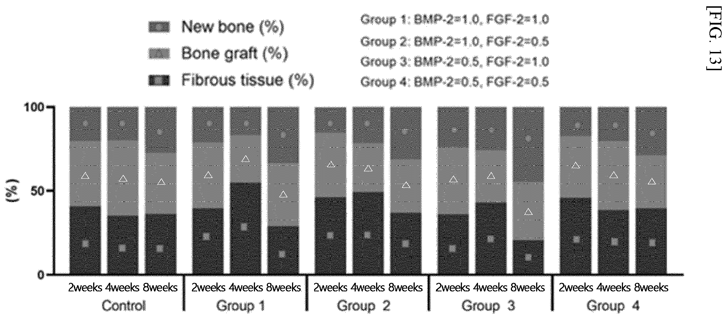

Excluding new bone and grafted bone, the remaining defects occurred on fibrous tissues. FIG. 13 is a graph showing how the three compositions change in each group depending on the treatment period. As the healing period progressed in all groups, the amount of new bone increased and the amount of bone graft material decreased.

### (4) Histological results (Russell-Movat Pentachrome staining)

### Experiment A

### 1) Histological sample analysis 2 weeks after healing

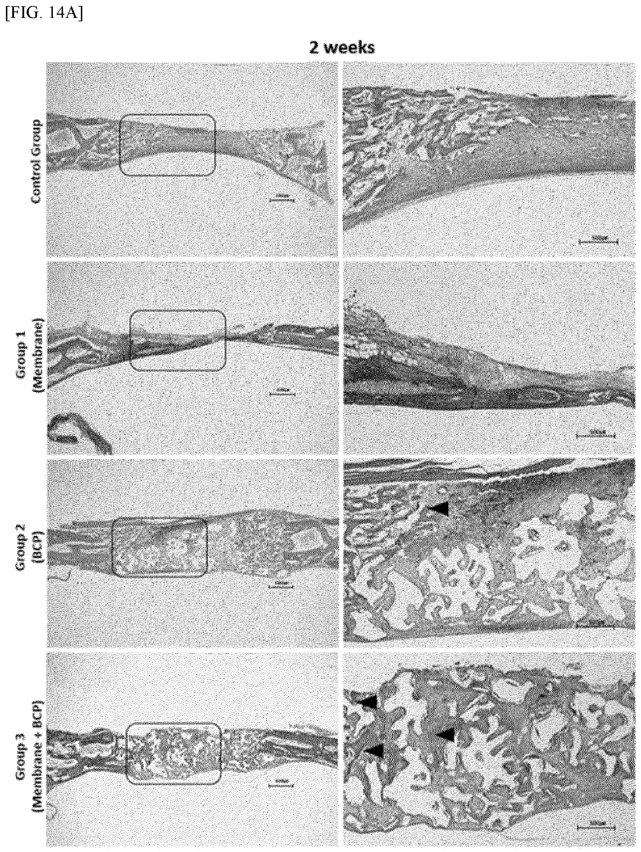

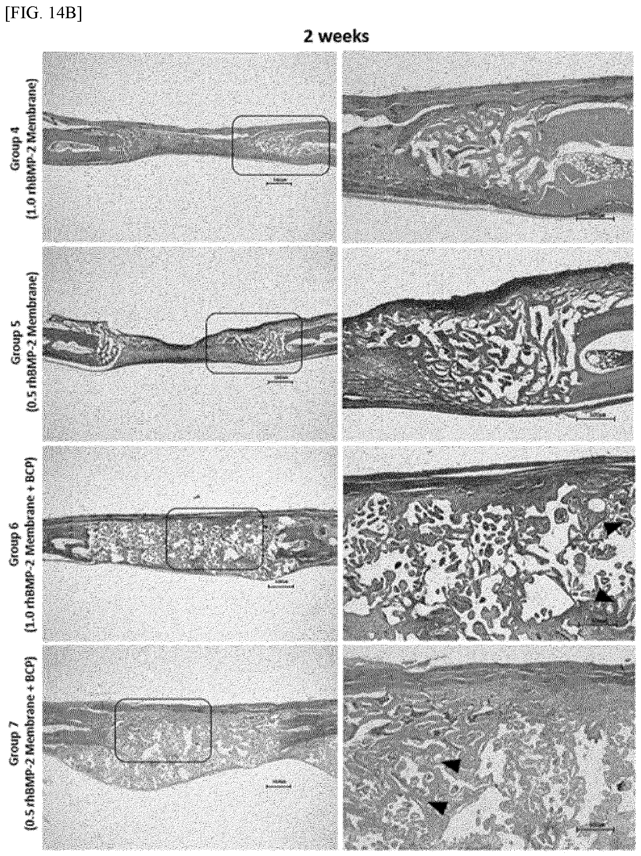

FIG. 14 shows photographs of Pentachrome-stained tissue specimens from the control and experimental groups after two weeks of healing. In most groups, the boundary between existing bone and new bone on both sides was clear. Experimental groups 2, 3, 6 and 7 all had BCP, so that new bone was formed around the existing bone and collagen fibers were formed around the bone graft material. In experimental groups 6 and 7, new bone grew from the bottom of the membrane to the center of the defect. However, this new bone was not mature, and many collagen fibers were formed on the top and around the bone graft material.

### 2) Histological sample analysis 4 weeks after healing

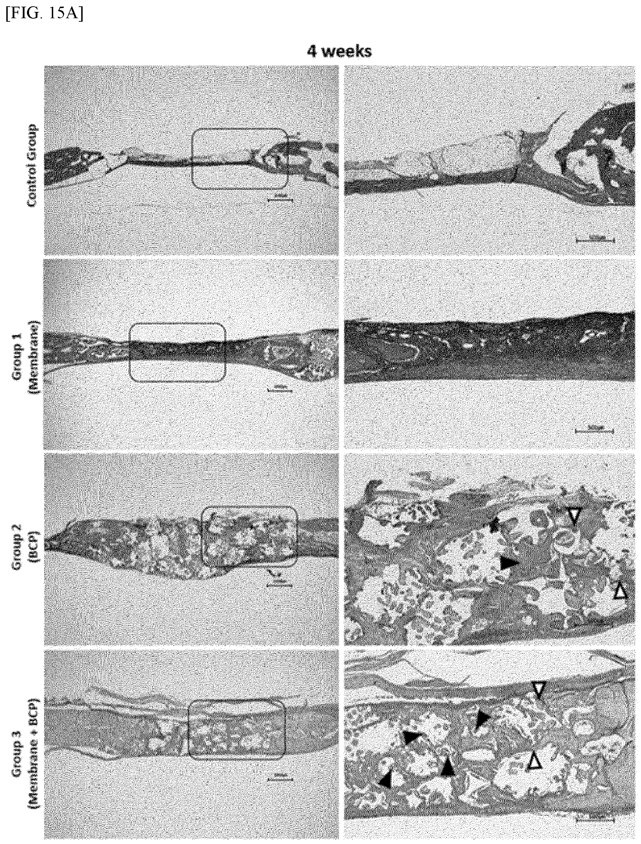

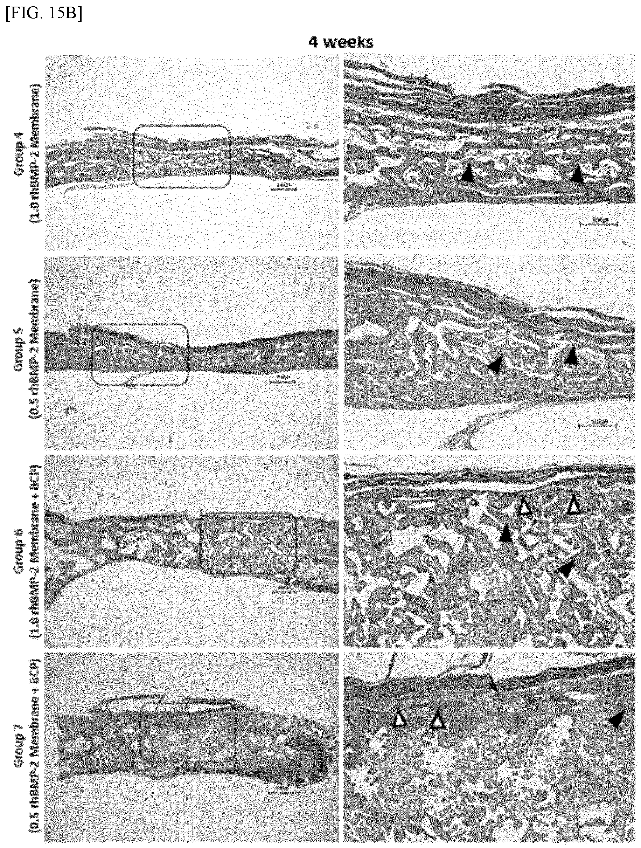

FIG. 15 shows photographs of Pentachrome-stained tissue specimens from the control and experimental groups 4 weeks after healing. In experimental group 3, mature new bone was formed around the existing bone, and new immature bone was found only inside the graft at the defect site. In experimental groups 4 and 5, thin new immature bone was also formed in the center of the defect. In experimental groups 6 and 7, mature new bone was observed in the upper part, but was not completely connected. In most groups, the new bone at the defect border and on the upper side became thicker and more mature than at 2 weeks, and the boundary between new bone and existing bone was relatively clear.

### 3) Histological sample analysis 8 weeks after healing

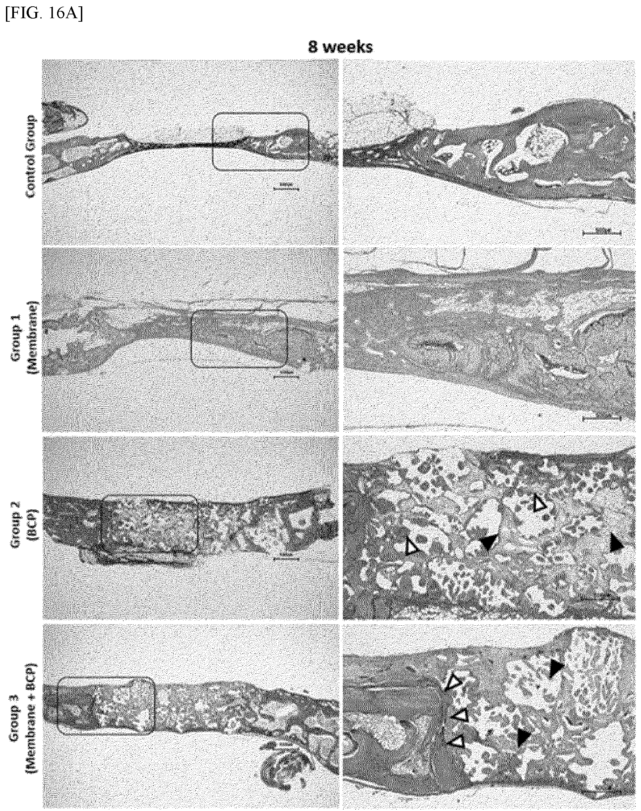

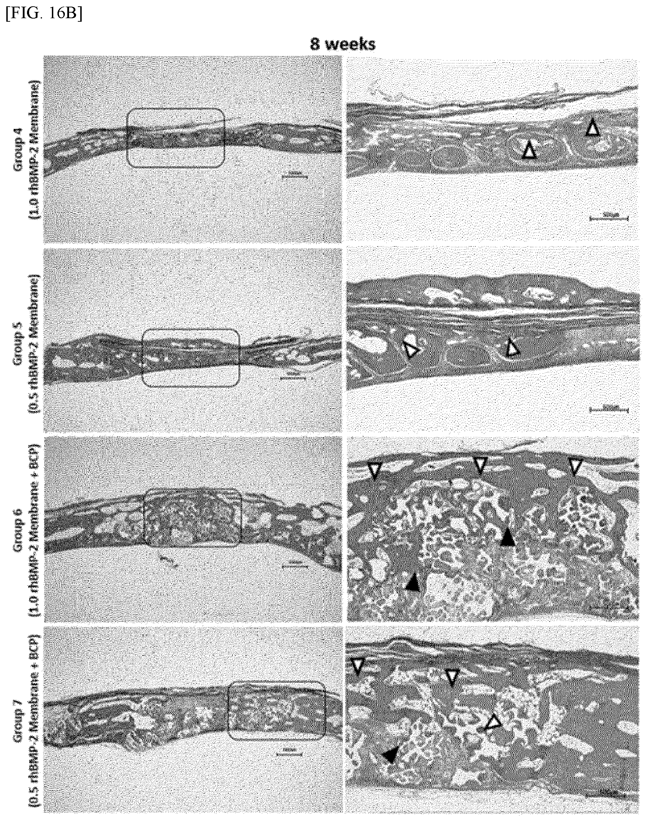

FIG. 16 shows photographs of Pentachrome-stained tissue specimens from the control and experimental groups after 8 weeks of healing. In all groups, new bone was histologically more mature than in the past, and the boundary between new bone and existing bone was unclear. In experimental groups 2 and 3, mature new bone was observed around the existing bone, but inside the defect area, new bone and collagen fibers were formed only around the bone graft material. In experimental groups 4 and 5, partially mature new bone was formed in the center of the defect. In experimental groups 6 and 7, mature new bone was connected from the existing bone to the center of the defect. Thin new bone was formed in the center of the defect.

### Experiment B

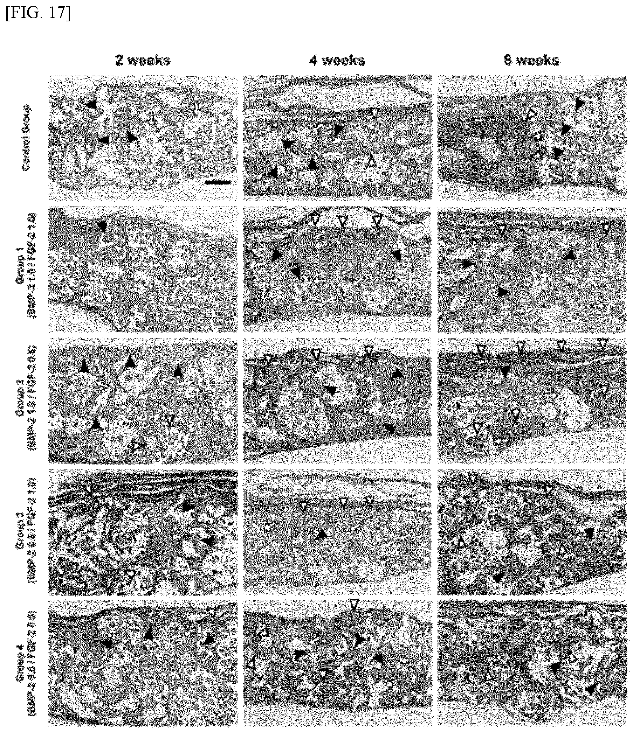

FIG. 17 shows images of pentachrome-stained tissue samples from the control and experimental groups to visualize the maturity of new bone. Mature bone stains from yellow to red, while immature bone stains green.

Two weeks after transplantation, new bone (black arrowhead) was observed near the existing bone in the control group, and collagen fibers were observed around the bone graft material (white arrow) in the pentachrome-stained specimen. In the experimental group, mature new bone (white arrowhead) was observed around the existing bone, and new bone (black arrowhead) was observed on the top and inside of the bone graft material. This new bone was not yet mature, and many collagen fibers were observed on the top and around the bone graft material (white arrows).

Four weeks after transplantation, in the control group, mature new bone (white arrowhead) was observed around the existing bone, and immature new bone (black arrowhead) was observed only inside the bone graft (white arrow) in the defect area. In the experimental group, similar to the control group, mature new bone (white arrowhead) was observed around the existing bone, but these bones were observed alone in the topore derived from the existing bone. The bones were not completely connected. As in the control group, new immature bone (black arrowhead) was observed inside the bone graft material.

Eight weeks after transplantation, in the control group, mature new bone (white arrowhead) was observed around the existing bone, but inside the defect, new bone (black arrowhead) and collagen fibers were observed only around the bone graft material (white arrow). The experimental group had continuous mature new bone (white arrowhead) that completely sealed the upper part of the defect, but the existing bone became thinner toward the center of the defect. No immature new bone was observed in the lower part of the defect in the experimental group.

### (5) Immunohistochemical results (anti-osteocalcin antibody staining)

### Experiment A

### 1) Tissue sample analysis 2 weeks after healing

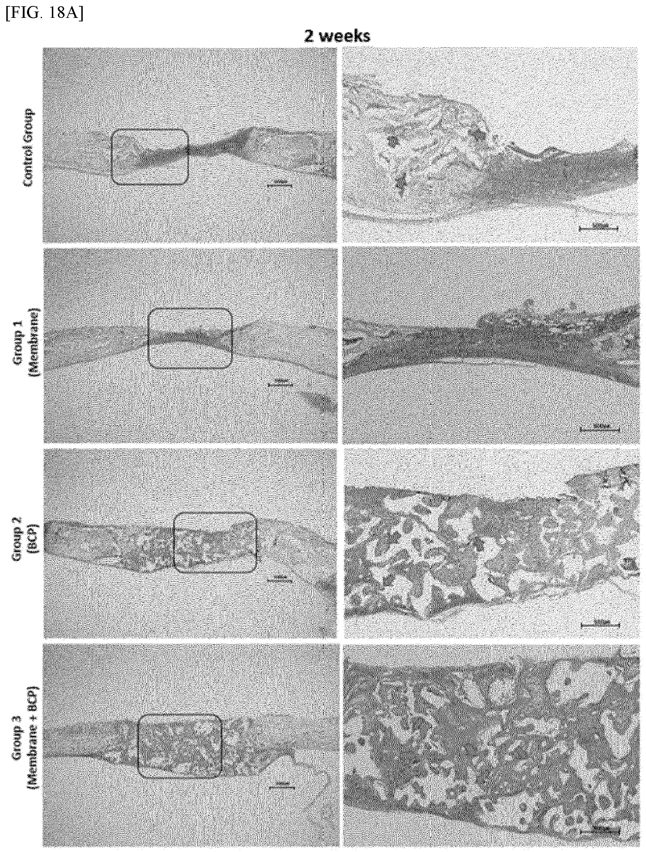

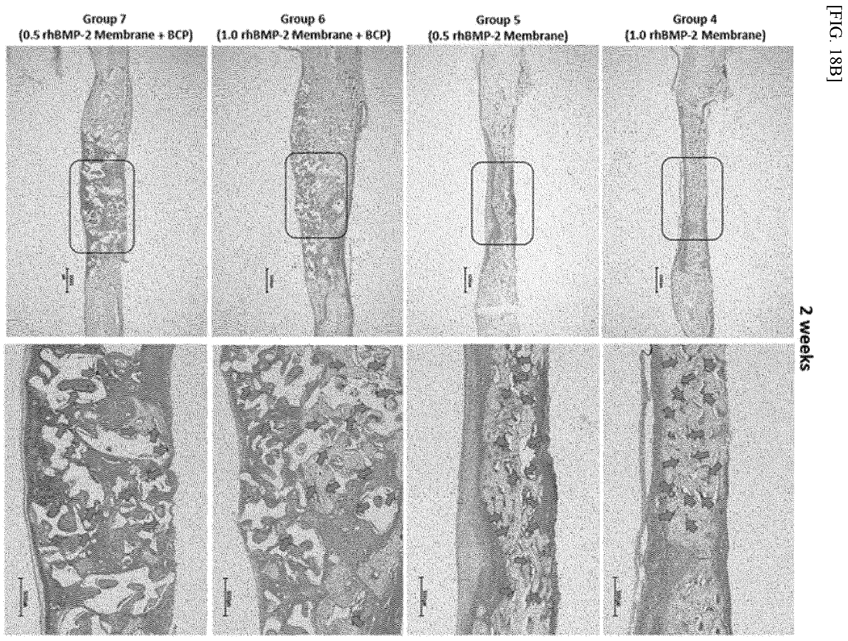

FIG. 18 shows photographs of OCG3-stained tissue specimens from the control and experimental groups two weeks after healing. In the control group and experimental group 1, only a small amount of osteocalcin was observed near the border of the defect. In experimental groups 2 and 3, osteocalcin was also found near the bone graft material in the center of the defect, but the amount thereof was smaller than that in experimental groups 4 to 7. In experimental groups 2, 3, 6 and 7, the osteocalcin was observed around the bone graft material. However, the expression of osteocalcin was higher in bone graft material around the existing bone than in the center of the defect.

### 2) Histological sample analysis 4 weeks after healing

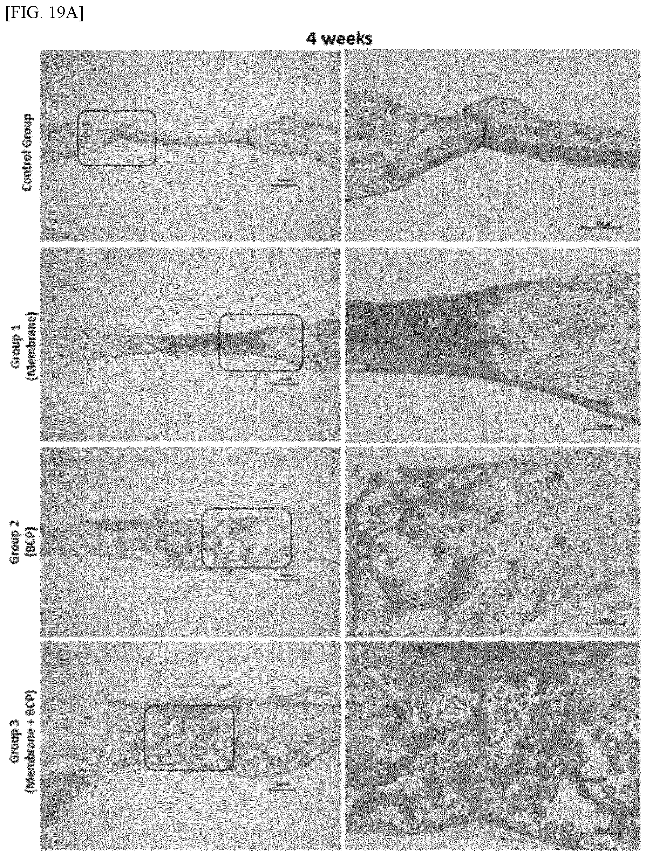

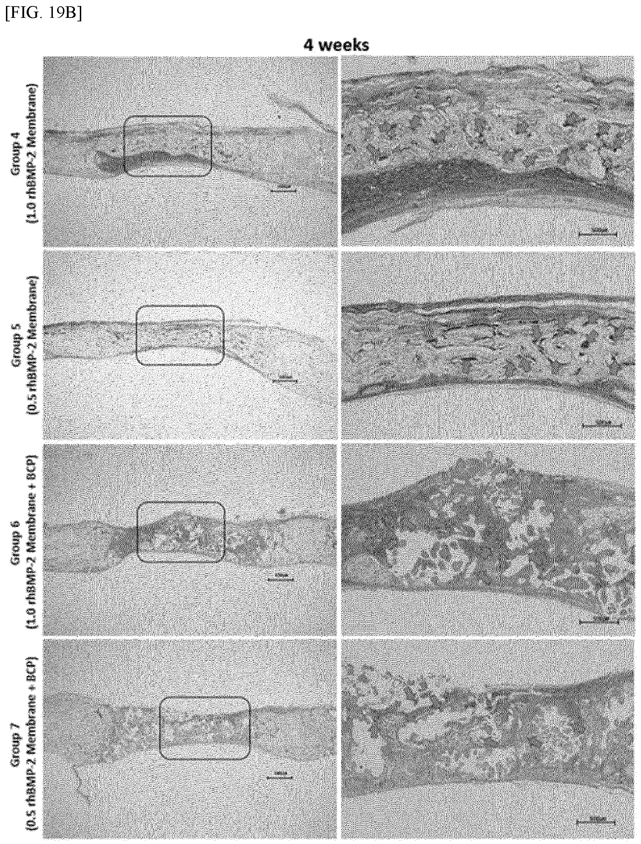

FIG. 19 shows images of OCG3-stained tissue specimens from the control and experimental groups 4 weeks after treatment. In all groups, more osteocalcin was found in the center of the defect than after 2 weeks, but not in the dura mater of the defect. In the control group and experimental group 1, osteocalcin was found only near the defect border, which was similar to the situation 2 weeks after healing. Groups 2 and 3 had more osteocalcin than after 2 weeks, but similar to after 2 weeks, there were many areas of osteocalcin expression around the bone graft material.

### 3) Histological sample analysis 8 weeks after healing

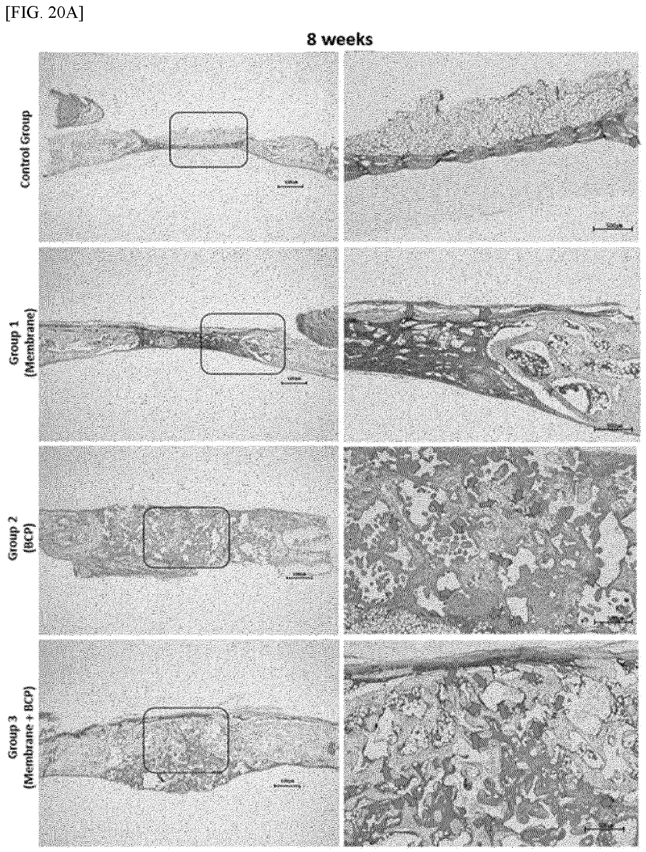

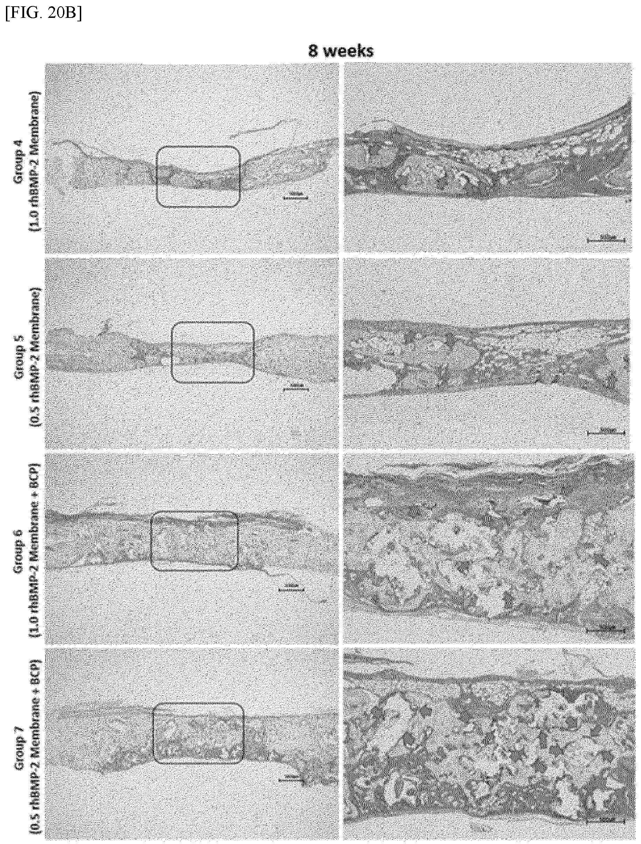

FIG. 20 shows images of OCG3-stained tissue samples from the control and experimental groups 8 weeks after treatment. In the control group, osteocalcin was limited to the border of the defect and was in a smaller amount than in the other groups. In experimental group 1, osteocalcin was detected up to the upper central part of the defect. In experimental group 6 where new bone completely filled the upper part of the defect, osteocalcin was observed near the internal bone graft material, not under the upper part of the defect. More osteocalcin was found in experimental groups 6 and 7 as compared to the healing period of 2 or 4 weeks.

### Experiment B

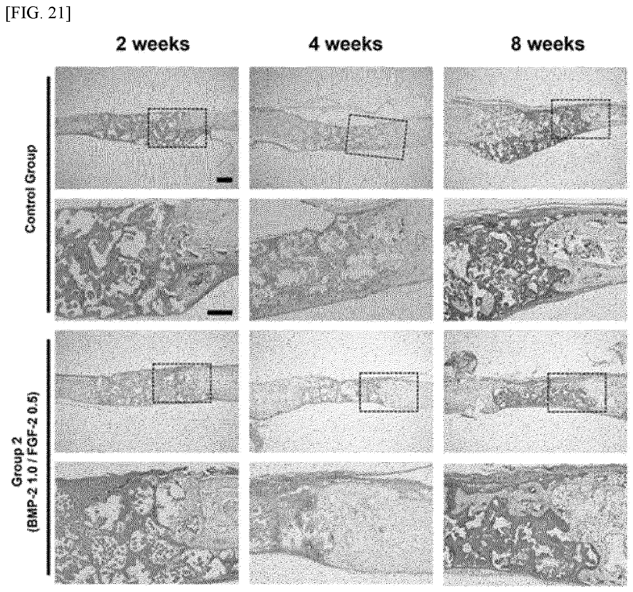

As shown in FIG. 21, osteocalcin was observed around the bone graft material in both the control group and experimental group 2 two weeks after transplantation. However, the expression of osteocalcin was higher in bone graft material than in existing bone.

The 4-week control group showed similar osteocalcin expression sites to the 2-week group. The 4-week experimental group showed a different osteocalcin expression site from that of the 2-week experimental group. These were mainly observed toward the collagen membrane, and when the upper part was sutured, they were located near the bone graft material in the center of the defect, not directly below the new bone as in experimental group 2 in FIG. 21.

8 weeks after transplantation, in the control group, similar to the 2-week and 4-week groups, osteocalcin was observed near the bone graft material around the existing bone, but there was no significant difference in the defect. In the 8-week experimental group, the expression of osteocalcin was observed, and when new bone under the collagen membrane filled the defect, osteocalcin was observed near the internal bone graft material rather than the mature covered bone on the upper part.

## Claims

1. A bone graft material, comprising:
a shielding film that covers an upper surface of a bone defect and separates the bone defect from an external environment; and
a filler that contacts a lower surface of the shielding film and fills the bone defect,
wherein the shielding film is impregnated with a BMP-2 (bone morphogenetic protein-2) solution.

2. The bone graft material according to claim 1, wherein a concentration of the BMP-2 solution is 0.1 to 1.0 mg/ml.

3. The bone graft material according to claim 1, wherein the filler is impregnated with any one selected from the group consisting of fibroblast growth factor (FGF), fibroblast growth factor 2 (FGF-2), platelet-derived growth factor (PDGF), vascular endothelial growth factor (VEGF), transforming growth factor (TGF-beta1), epidermal growth factor (EGF), insulin-like growth factor (IGF), stem cells and exosomes.

4. The bone graft material according to claim 1, wherein the filler is impregnated with 0.5 to 1.5 mg/ml of FGF-2 solution.

5. The bone graft material according to claim 1, wherein the shielding film has a thickness of 0.1 to 0.5 mm.

6. The bone graft material according to claim 1, wherein the shielding film has a porous structure with BMP-2 contained in pores.

7. The bone graft material according to claim 1, wherein the shielding film is made of any one selected from the group consisting of collagen, gelatin, alginate, demineralized bone matrix, hyaluronan, deproteinized bone matrix, chitosan, β-tricalcium phosphate, bioglass, hydroxyapatite, calcium phosphate, expanded polytetrafluoroethylene, polymethylethyl methacrylate, polylactic acid, polyglycolic acid, polyurethane, polylactide-co-glycolide, or a combination thereof.

8. The bone graft material according to claim 1, wherein the filler is made of any one selected from the group consisting of hydroxyapatite, tricalcium phosphate, biphasic calcium phosphate, bioglass, calcium sulfate, calcium carbonate, calcium phosphate, polyurethane acrylate, polyvinyl alcohol, polyethylene, polypropylene, polyethylene glycol, polylactad-co-glycolide, polycaprolactone, polylactic acid, polyglycolic acid, carboxylic acid, chitosan, isoleucine, ethyl ester, gelatin, collagen, and a combination thereof.

9. A bone formation method, comprising:
filling a bone defect with a filler; and
forming a shielding film impregnated with a BMP-2 solution on an upper surface of the filler in order to separate the bone defect from an external environment.

10. The bone formation method according to claim 9, wherein a concentration of the BMP-2 solution is 0.1 to 1.0 mg/ml.

11. The bone formation method according to claim 9, wherein the filler is impregnated with any one selected from the group consisting of fibroblast growth factor (FGF), fibroblast growth factor 2 (FGF-2), platelet-derived growth factor (PDGF), vascular endothelial growth factor (VEGF), transforming growth factor (TGF-betal), epidermal growth factor (EGF), insulin-like growth factor (IGF), stem cells and exosomes.

12. The bone formation method according to claim 9, wherein the filler is impregnated with 0.5 to 1.5 mg/ml of FGF-2 solution.

13. The bone formation method according to claim 9, wherein the shielding film has a thickness of 0.1 to 0.5 mm.

14. The bone formation method according to claim 9, wherein the shielding film has a porous structure with BMP-2 contained in pores.

15. The bone formation method according to claim 9, wherein the shielding film is made of any one selected from the group consisting of collagen, gelatin, alginate, demineralized bone matrix, hyaluronan, deproteinized bone matrix, chitosan, β-tricalcium phosphate, bioglass, hydroxyapatite, calcium phosphate, expanded polytetrafluoroethylene, polymethylethyl methacrylate, polylactic acid, polyglycolic acid, polyurethane, polylactide-co-glycolide, or a combination thereof.

16. The bone formation method according to claim 9, wherein the filler is made of any one selected from the group consisting of hydroxyapatite, tricalcium phosphate, biphasic calcium phosphate, bioglass, calcium sulfate, calcium carbonate, calcium phosphate, polyurethane acrylate, polyvinyl alcohol, polyethylene, polypropylene, polyethylene glycol, polylactad-co-glycolide, polycaprolactone, polylactic acid, polyglycolic acid, carboxylic acid, chitosan, isoleucine, ethyl ester, gelatin, collagen, and a combination thereof.
